# EUROPEAN PATENT APPLICATION

(11) **EP 4 520 366 A1**
(43) Date of publication of application: **12.03.2025**
(21) Application number: 24166737.7
(22) Date of filing: 27.03.2024
(51) Int. Cl.: A61M 5/20, A61M 5/32

(54) **ATTACHMENT AND LOCKING OF RESERVOIR UNIT OF INJECTION DEVICE**

(30) Priority: 07.09.2023 EP 23195978
(71) Applicant: Ypsomed AG, 3401 Burgdorf (CH)
(72) Inventor: Scheurer, Simon, 3006 Bern (CH); Schüpbach, Simon, 3400 Burgdorf (CH)
(74) Representative: Kleiner, Stefan

(57) **Abstract**

The invention relates to a method for releasably attaching and locking a reservoir unit (2) to a drive unit (3) of an injection device (1). The injection device includes a cap (10) attachable to a distal end portion of the reservoir unit (2). The latter is adapted to hold the reservoir (15) and includes an outer cover (20) with a lateral opening (22) and a release member (32) movable relative to the cover wherein the release member (32) can be in a locking position in which the cap (10) is locked to the reservoir unit (2) and in a release position in which the cap is released. The drive unit (3) comprises a housing including an axial opening (81), a clamping arm (61) movable relative to the housing and having a connecting portion (62) on a free end, an actuation arm (66) movable relative to the housing and having a locking portion (67) on a free end and a trigger member movable along the longitudinal axis.

## Description

### FIELD OF THE INVENTION

The present invention relates to a releasable attachment and locking of a reservoir unit to a drive unit of an injection device for injecting, delivering, administering, infusing or dispensing substances and/or liquids such as insulin, hormone preparations or vaccines. The drive unit comprises a housing including an axial opening for accommodating the reservoir unit a clamping arm for holding a protrusion of the reservoir unit and an actuation arm for locking the reservoir unit to the drive unit.

### BACKGROUND OF THE INVENTION

A variety of diseases exist that require regular treatment by subcutaneous administration of a medicament, and several drug delivery devices have been developed to support a patient in accurately and controllably delivering an amount of drug in a self-administration process. Delivery devices include injection devices that are removed from the injection site after each medication event or drug delivery process, as well as infusion devices with a cannula or needle that remains in the skin of the patient for a prolonged period of time.

By way of example, diabetes may be treated by self-administration of insulin or its derivatives with the help of multi-variable-dose insulin injection pens. An injection pen device generally has an elongate device body defining a longitudinal main device axis. An automatic injection device has a motor or a drive spring for biasing a plunger rod and shifting a piston in a container barrel, wherein the drive spring may have to be charged or strained manually prior to injection of a dose. A manually powered delivery drive requires a user to manually provide the energy to move the piston, for instance by applying a distal force component to the injection device.

The medicament dose to be injected may typically be manually selected by turning a dosage knob and observing the actual dialed dose from a dose window or display of the insulin pen. A dose is dispensed by inserting the needle into a suited portion of human skin and by moving the piston manually or by pressing a release button of an automatic injection device. Automatic injection devices may comprise an electronic dose dial mechanism to automatically set a dose.

Also known is the use of autoinjectors with syringes. Autoinjectors usually comprise a body for housing a syringe as well as a drive mechanism in order to move the plunger of the syringe upon actuation of the autoinjector. The drive mechanism typically comprises a source of drive, such as an electric motor or a mechanical strong spring for moving a transfer element, for example a rod, which acts on the plunger of the syringe. The prefilled syringe has a needle or cannula that is permanently attached to a first end of a syringe barrel or reservoir that is sealed at the opposing end by the plunger.

For safety and hygiene reasons it is desirable that the needle does not protrude from a housing of the autoinjector except for the time when the needle is used for injection of a medicament. Thus, either the autoinjector moves the needle out of the housing for the injection and back into the housing after injection or the autoinjector includes a needle guard which may be moved to unsheathe the needle for injection and which may be moved back to a needle covering position after the injection.

The majority of autoinjectors are configured as single use devices which incorporate both the syringe and the drive mechanism in the same housing. Such devices are usually disposable for hygiene reasons.

Disposable autoinjectors comprising an electric actuator or an electronic control require a source of energy which is usually in the form of a battery. However, in this case, the autoinjectors should not be disposed in the regular waste but have to be subjected to special disposal or to recycling, which is an additional burden to the patient. Further, disposing a battery, motor and/or electronics after a single use is a waste of resources and increases the costs of the therapy.

In order to account for a need to handle and dispose the autoinjector parts differently semi-reusable autoinjectors have been developed. Such autoinjectors typically comprise a reusable drive unit as well as a disposable syringe unit which may be releasably coupled or attached to the drive unit. The drive unit usually includes the drive mechanism and electronics whereas the syringe unit includes the syringe with the needle and a needle cover sleeve. The user can thus discard the syringe unit when it is empty or after use and can load the drive unit with a new syringe unit for a new upcoming injection.

WO 2012/038721 A1 discloses a semi-reusable autoinjector with a syringe unit including a syringe and with a drive unit including a torsion spring and a gear to automatically dispense the drug from the syringe. The drive unit comprises an opening to accommodate the syringe unit. In a bottom plate in the opening two forwardly projecting lugs are designed to enter recesses in the syringe unit housing upon insertion of the syringe unit to unlatch a rearward collar of a syringe shield. This allows the syringe shield to move to a retracted position.

WO 2010/046569 discloses a reusable autoinjector with a syringe unit to be coupled to a drive unit by a bayonet connection. Once a cam of the drive unit is inserted in a slot in the syringe unit housing a cover sleeve is unlocked and can be moved to unsheathe an injection needle of the syringe unit.

WO 2012/145685 discloses a reusable autoinjector with a disposable or single-use cassette and a reusable drive unit. The cassette includes an outer housing, an inner sleeve, a shield remover, a movable cover and a lock cap locking the syringe to the inner sleeve.

WO 2021/069106 discloses an autoinjector comprising a syringe unit and a drive unit. When an axially protruding locking pin of the drive unit is pushed through in a pin opening in the syringe unit, it encounters a syringe holder arm. This results in a deflection of the holder arm, which in turn unlocks a needle cover sleeve and allows it to move in the proximal direction.

### DESCRIPTION OF THE INVENTION

It is an objective of the invention to provide for a simple as well as a reliable attachment and locking of a reservoir unit to a drive unit of an injection device.

This objective is achieved by a method and use according to the independent claim. Preferred embodiments are evident from the dependent claims.

The invention relates to a method for releasably attaching and locking a reservoir unit to a drive unit of an injection device. In an attached state the injection device is adapted to dispense a liquid drug through an injection needle. However, the invention and the claimed method do not relate to the injection itself and thus have no functional link to any therapeutic effect. The claimed method exclusively relates an assembly (attachment and locking) of components of the injection device well before an injection or interaction with a human body.

The injection device according to the invention includes a reservoir unit, a drive unit and a cap attachable to a distal end portion or distal end of the reservoir unit. The reservoir unit is adapted to hold the reservoir and the reservoir unit includes
- an outer cover (preferably cylindrically shaped) with a lateral opening (in a side wall of the cylinder);
- preferably or optionally a radially extending protrusion extending from a center;
- a release member movable relative to the cover (and relative to a reservoir holder) wherein the release member can be in a locking position in which the cap is held on and lock to the reservoir unit and in a release position in which the cap is released and not locked to the reservoir unit such that it can be removed.

The drive unit of the injection device according to the invention comprises
- a housing defining a longitudinal axis and including an axial opening adapted to accommodate at least a portion of the reservoir unit for attaching and locking the reservoir unit to the drive unit;
- preferably or optionally a clamping arm movable relative to the housing and comprising a connecting portion on a free end of the clamping arm;
- an actuation arm movable relative to the housing and comprising a locking portion on a free end of the actuation arm, the actuation arm being distinct of the clamping arm and
- a trigger member movable along the longitudinal axis relative to the housing;
wherein the method comprises the steps of
- Inserting at least a proximal portion of the reservoir unit into the axial opening;
- Optionally coupling, by the insertion, the connecting portion to the protrusion to releasably attach the reservoir unit to the drive unit such that the reservoir unit cannot fall off but is not yet locked to the drive unit;
- Moving the trigger member, by a drive of the drive unit, along the longitudinal axis to encounter or to keep engagement with the actuation arm;
- Moving the actuation arm, by the longitudinal trigger member movement, from a radial outer position into a radial inner position thereby inserting the locking portion into the lateral opening such that locking portion, such that locking portion prevents a distal retraction movement of the reservoir unit and locks the reservoir unit to the drive unit and
- Additionally engaging, by locking portion in its inner position, the release member and thereby moving the release member into its release position or hold the release member in its release position such that the cap can be removed by the user from the reservoir unit.

The method according to the invention allows to couple or attach and subsequently lock the reservoir unit to the drive unit. With a simple and preferably exclusive longitudinal movement (manually or automatically) of the trigger member relative to the drive unit housing the reservoir unit is attached and locked to the drive unit such that it cannot be removed anymore without unlocking.

In contrast to known approaches which focus on a radial coupling and release (in a plane perpendicular to the longitudinal axis) according to the invention the trigger member is movable along the longitudinal axis. As the trigger member does not move in radial direction the dimensions of the drug delivery device in radial direction can be reduced compared to known approaches.

Moreover, the longitudinal movement of the trigger member releases the cap such that the cap can be removed, either manually by a user force or by means of a biasing element.

The release member can be used to redirect or to reroute a (automatic) release movement of the injection device or a manual release movement of the user to automatically release the cap or unlock the cap before an injection.

In the present description the term coupling or attaching means that reservoir unit is held on the drive unit and cannot fall of. However, if the user pulls the reservoir unit in distal retraction direction relative to the drive unit the releasable connection between drive unit and the reservoir unit and in particular between the connecting portion and the protrusion is released or disconnected and the reservoir unit can be separated from the drive unit.

In contrast thereto, the term locking means in the present context that the reservoir unit is connected to the drive unit and cannot be separated in non-destructive manner. Namely, if the locking portion is inside the lateral opening the reservoir unit is locked to the drive unit by form-fit engagement and cannot be removed by simply pulling the reservoir unit. A distal retraction movement cannot unlock the reservoir unit from the drive unit. The reservoir unit is locked to the drive unit unless the locking portion is moved out of the lateral opening and hence moved from its inner position to its radially outer position.

The injection device may be either a manually actuated or an automatic injection device. Automatic devices or autoinjectors typically include automatic drive means such as, for example, an elastic element (for example a pre-tensioned spring) or an electric motor to drive a dispensing member to dispense the liquid drug form the reservoir. Manual injection devices include a manual drive such as a dispensing button that has to be pressed by the user and by a human force to move the dispensing member in dispensing direction.

The reservoir of the injection device may include a prefilled syringe which has a needle or cannula that is permanently attached to a first end of a syringe barrel and that is sealed at the opposing end by a plunger. Alternatively, the reservoir may be a cartridge comprising a needle mounting portion adapted to be connected to a needle assembly prior injection.

The injection device may comprise further components such as a shield or locking mechanism, for example, to prevent unintentional access to an injection needle. The reservoir unit is preferably disposable and is discarded after use. The drive unit is preferably reusable meaning that it can be used for several injection and with different reservoir units. In this case, the injection device may be a semi-reusable injection device and in particular a reusable autoinjector (also named as semi-disposable autoinjector) having the reusable drive unit and the disposable reservoir unit including a syringe or cartridge with the drug.

In these semi-reusable injection device concepts, the reservoir unit is typically not operational without a reusable drive unit of the injection device. To prepare the injection device for an injection the user has to attach or to couple the reservoir unit to the drive unit.

The trigger member is adapted to lock or unlock the reservoir unit to the drive unit. The trigger member may be, for example, a sleeve or a nut movable relative to the housing. The trigger member may be moved manually by a user force or automatically by an automatic drive such as an electric motor or a preloaded spring member. In a preferred embodiment the trigger member is arranged inside a housing of the drive unit. The trigger member is thus not arranged in an injection device cap or in an external device. In a further preferred embodiment, the trigger member is sleeve-shaped and coaxially arranged to a reservoir inside the housing. This allows for a space-saving design.

Furthermore, the trigger member can actuate the actuation arm. That means displacing the trigger member along the longitudinal axis moves the actuation arm between an outer position and a radially inner position. In its inner position the actuation arm abuts a distal surface of the reservoir unit and thus prevents a movement of the reservoir unit in distal direction and hence prevents a removal of the reservoir unit from the drive unit. That is, a locking portion of the actuation arm encounters or contacts the distal surface of the reservoir unit and thus stops or blocks a possible retraction movement.

Additionally, in its inner position the actuation arm actuates the release member such that the release member moves in its release position or is kept in the release position such that it releases the cap. The cap can then be removed from the reservoir unit. Therefore, the actuation arm fulfills two functions it locks the reservoir unit to the drive unit and moves or holds the release member in its release position.

The drive unit preferably includes a clamping arm which is adapted to hold the reservoir unit by its connecting portion, but the reservoir unit is not locked to the drive unit by the clamping arm. That means the user can still pull out the reservoir unit from the drive unit housing by overcoming a holding force. The connecting portion may include a snap-fit element or form-fit element adapted to engage the protrusion.

The release member is adapted to selectively hold or release the cap on the reservoir unit. The release member may be, for example, a mechanical switch or bistable element such as a relay, rocker or seesaw. In a locking position the release member holds and locks the cap on the reservoir unit, for example, by a holding arm or clamp. In a preferred embodiment the cap is hold via intermediate member (for example a rocker or connecting element between the holding element and the release member). The intermediate member can be actuated or displaced by the release member when it is moved between its locking position and its release position. In its release position the cap is released and not hold any more by the release member, e.g. the holding arm or camp is moved away from the cap allowing the user to pull off the cap.

The reservoir unit comprises a lateral opening in a sidewall or in side surface and a protrusion extending radially. The lateral opening and the protrusion are preferably rotationally aligned to each other meaning that the opening and protrusion are located on a common axis parallel to the longitudinal axis. The lateral opening is preferably arranged distally to the protrusion. Furthermore, the lateral opening may be arranged in a middle portion of the reservoir unit and the protrusion may be located in a proximal end portion which facilitates the coupling with the connecting portion.

The protrusion may be formed by a reservoir holder or support structure of the reservoir unit. In a preferred embodiment the cover comprises a recess for the protrusion and the protrusion protrudes from the cover such that the connecting portion can be easily coupled to the protrusion upon insertion of the reservoir unit into the axial opening of the drive unit.

The outer cover may be fixed relative to a reservoir holder or relative to a supporting structure or alternatively, it may be movable, preferably along the longitudinal axis. In the latter case the lateral opening and the recess may have an elongated shape along the longitudinal axis allowing the connecting portion and the locking portion to travel within the opening and the recess, respectively, when the cover is moved relative to the holder.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances

The term "distal" is meant to refer to the direction or the end of the injection device carrying an injection needle or an injection cannula, whereas the term "proximal" is meant to refer to the opposite direction or end pointing away from the needle or cannula.

The term "injection system" or "injector" refers to a device that is removed from the injection site after each medication event or drug delivery process, whereas the term "infusion system" refers to a device with a cannula or needle that remains in the skin of the patient for a prolonged period of time, for example, several hours.

Preferably, the insertion of the insertion of the proximal portion of the reservoir unit into the drive unit includes a coupling of the connecting portion of the movable clamping arm of the drive unit to the radially extending protrusion of the reservoir unit to releasably couple the reservoir unit to the drive unit.

In a preferred embodiment the connecting portion includes a sloped surface contacting the protrusion upon insertion such that the clamping arm radially deflects when the reservoir unit is moved along the longitudinal axis and moved into the axial opening of the drive unit for attachment. The sloped surface thus facilitates the coupling between the protrusion of the reservoir unit and the connecting portion. The sloped surface is preferably a distally oriented surface and sloped or angled with respect to the longitudinal axis of the injection device.

Preferably, the connecting portion includes a clamp or recess and wherein during insertion and attachment the protrusion enters the clamp or recess and establishes a releasable snap-fit connection to couple connecting portion to the protrusion. The clamp may be biased in a holding position, for example, by a spring or elastic element. The clamp thus engages the protrusion of the reservoir unit upon insertion and reliably holds the reservoir unit in an attached position. However, when the clamp is engaged to the protrusion the reservoir unit is not yet locked and the user can pull the reservoir unit out of the drive unit against a holding force of the clamping arm.

In a preferred embodiment the clamp or recess includes a proximally oriented holding surface encountering or engaging the protrusion when the reservoir unit is attached to the drive unit. The holding surface is radially offset of a center of rotation of the clamping arm such that the clamping arms radially deflects when a force acts onto the holding surface along the longitudinal direction and in distal direction.

The radially offset located holding surface ensures that the connecting portion and the whole clamping arm reliably deflects or moves out of engagement and thus the protrusion is reliably released from the clamp or recess when reservoir unit is moved distally, e.g. when the user pulls the reservoir unit out of the drive unit. In the same way, the offset of the holding surface facilitates deflection upon insertion.

Preferably, the protrusion has a rectangular-shaped cross section in a plane parallel to the longitudinal axis. The rectangular shape may help to align the reservoir unit relative to the drive unit. Alternatively, the protrusion may have a circular or oval cross section.

The locking portion of the actuation arm includes preferably a radially inwards protruding cam comprising a proximally oriented locking surface. When the reservoir unit is attached and when it is moved distally the proximal locking surface engages or abuts the distal surface of the reservoir unit to lock the reservoir unit to the drive unit. That means the user cannot retract and remove the reservoir unit for the drive unit when the actuation arm is in its inner position and when the locking surface encounters the distal surface of the reservoir unit.

The locking portion with the cam provides a quick and reliable locking of the reservoir unit to the drive unit.

Preferably, the lateral opening has an elongated form extending along the longitudinal axis and wherein the distally oriented surface defines a proximal end of the lateral opening.

That means when the actuation arm is in its inner position the locking portion engages and contacts the proximal end of the lateral opening and thus safely locks the reservoir unit to the drive unit after the attachment.

The drive unit preferably includes two oppositely arranged camping arms and preferably two oppositely arranged actuation arms. Correspondingly, the reservoir unit comprises two oppositely arranged protrusions and preferably two oppositely arranged lateral openings such that two connecting portions engage the two protrusions and two locking portions can be entered into the two lateral openings when the reservoir unit is attached to the drive unit.

The two arms allow to reliably hold the reservoir unit when it is attached to the drive unit.

Preferably, the outer cover is a needle cover which is the outermost part of the reservoir unit. The needle cover may be movable along the longitudinal axis relative to an injection device housing between a needle covering position, in which the needle is covered and a retracted position, in which the needle is exposed. That means the reservoir unit does not comprise any outer housing or shell but only the movable needle cover. This provides for a simple design.

The outer cover acts as a shield or cover to prevent unintentional access to the needle and may prevent injuries by the injection needle.

The needle cover may be implemented as shield or sleeve, or sleeve-shaped element adapted to cover or envelop a needle of a prefilled syringe or a carpule hold by the reservoir holder. The movement of the needle cover allows to switch between a safety state in which the needle is covered, and the needle does not protrude from a device housing and an injection state in which the needle is uncovered and exposes or protrudes from the housing and from the needle cover.

In a further embodiment the reservoir unit may comprise a lock element movable relative to the cover. The lock element may be adapted to block or hold the needle cover in the needle covering position. The lock element may be a deflectable, rotatable or shiftable element, for example in form of a ledge, arm, protrusion or knob. Further, the lock element may be a movable element of the needle cover or of a housing part adapted to contact, abut or engage the needle cover.

The lock element can be in the locking position and may be moved into an unlocking position, intermediate position, or end position. If the lock element is in the locking position it preferably abuts a stop surface of the needle cover or of a support or housing element to prevent the needle cover from being moved out of the covering position. If the lock element is not in the locking position it does not prevent the needle cover to be moved out of the covering position.

Preferably, the trigger member movement actuates the lock element. That means the trigger member can be brought into contact with the lock element either directly or indirectly via an intermediate member. In the latter case the trigger member contacts or engages the intermediate member which in turn contacts or engages the lock element. In this way, a movement of the trigger member is transferred to the lock element to move the lock element out of the locking position towards an unlocking position in which the lock element does not hinder or block the needle cover and the needle cover is movable out of the covering position towards a retracted position.

Optionally, the longitudinal shifting or movement of the trigger member can be transferred to a radial deflection or movement of the lock element, either directly or indirectly via an intermediate member.

In a preferred embodiment the reservoir unit further includes a deflectable holding member holding the cap on the distal end portion and wherein the release member is a rocker tiltable around a center which divides the rocker into a first and a second rocker portion. The first portion of the rocker may include an actuation surface that encounters the locking portion, in particular a cam of the locking portion, to tilt the rocker when actuated and thus moves the rocker around the center and wherein the second portion engages the holding member.

In a first or initial tilt position of the rocker the holding member can hold the cap on the distal end and in a second tilt position which is different from the first tilt position the second portion deflects the holding member such that the cap is released and can be removed from the distal end portion.

The rocker provides a reliable releasing or unlocking of the cap as the rocker can be in well-defined tilt positions. Namely, the rocker can be in a clearly defined and stable first, neutral or initial tilt position (e.g., in a shipping position) in which the second portion of the rocker does not deflect the holding member and thus the holding member can hold the cap onto the distal portion of the assembly. In the defined and stable second tilt position of the rocker the second rocker portion can engage and deflect the holding member such that the holding member no longer holds the cap in the initial cap position and thus the cap can be removed, either manually by a user force or by means of a biasing element.

By means of the rocker working as a seesaw one end portion of the rocker can be actuated, in particular pressed down or up or radially inwards or outwards and thus the other end portion of the rocker is moved in counter direction. The rocker can thus be used to redirect or to reroute a (automatic) release movement of a release member of the injection device or a manual release movement of the user.

The holding member is preferably adapted to hold the cap in place and to fix the cap on a distal end of the assembly and of the injection device. The deflectable holding member may be, for example, a clamp, arm, tongue or shell. Preferably, the holding member is elastically deformable such that it can be deflected to release the cap and then can deflect back to its original position.

The cap is released from the distal end portion if the holding member is in its second tilt position. The term "released" means the cap is free to be removed by the user from the distal end. Hence, the cap is not locked or otherwise hold. The term released does not mean that the cap is automatically moved or shifted off or pushed off by the release mechanism. Preferably, the cap remains on the distal end portion after release and has to be removed by the user.

Preferably, an axial movement of the trigger member relative to the housing moves the actuation arm with its locking portion to its radial inner position to encounter the actuation surface which moves the rocker from the first tilt position to the second tilt position.

Preferably, the rocker is tiltable around an axis perpendicular to the longitudinal axis. That allows to arrange the rocker in a space saving manner as in this orientation the rocker may extend along the longitudinal axis. Alternatively, the pivot axis may be parallel to the longitudinal axis. In this case the rocker is tiltable cross to the longitudinal device axis.

The invention further relates to a use of the reservoir unit adapted to hold the reservoir containing the liquid drug. The reservoir may comprise a protrusion and a lateral opening for attaching and locking the reservoir unit to the drive unit as described above.

The use concerns preferably a reservoir unit which is a syringe unit comprising a prefilled syringed with an injection needle permanently attached to a syringe barrel and wherein the syringe unit further includes a syringe holder adapted to immovably hold the syringe such that the syringe cannot move relative to the syringe holder before, during and after the injection.

In an alternative embodiment the use concerns a reservoir unit which is a cartridge unit comprising a cartridge with a needle mounting portion on a distal end for releasable attachment of an injection needle assembly and wherein the cartridge unit further includes a cartridge holder for immovably hold the cartridge.

The present disclosure further relates to a disposable reservoir unit adapted for releasable attachment to a reusable drive unit of an injection device for dispensing a liquid drug from a reservoir through an injection needle. The injection device includes a cap releasably attachable to a distal end portion of the reservoir unit. The reservoir unit includes a holding member adapted to hold the cap on the distal end portion and the reservoir unit further comprises:
- a reservoir holder for holding the reservoir containing the liquid drug, the reservoir holder defining a longitudinal axis;
- preferably or optionally a protrusion radially extending from the reservoir holder
- an outer cover being an outermost part and comprising a lateral opening adapted to accommodate a locking portion of an actuation arm of the drive unit when the reservoir unit is attached to the drive unit, preferably the opening being rotationally aligned with the protrusion and preferably positioned distally to the protrusion;
- a release member movable relative to the cover wherein the release member can be in a locking position in which the cap is held and locked by the holding member to the reservoir unit and wherein the release member can be in a release position in which the cap is released, wherein the release member is accessible through the lateral opening by the locking portion to switch the release member between the locking position and the release position.

Preferably, in a plane perpendicular to the longitudinal axis, the reservoir unit has an elliptical form defining a major axis and a minor axis and wherein the protrusions are located on the major axis.

The protrusion has preferably a radial height that increases from a first side of the protrusion to a center of the protrusion along the longitudinal axis thereby defining a first sloped surface. Furthermore, the protrusion may have a radial height that increases from a second side to the center of the protrusion along the longitudinal axis thereby defining a second sloped surface. The first and second sloped surface may facilitate a coupling with a connection portion of a clamping arm of the drive unit to attach the reservoir unit to the drive unit.

The protrusion has preferably a rectangular-shaped cross section in a plane parallel to the longitudinal axis wherein a dimension of the protrusion along the longitudinal axis is larger than a dimension perpendicular to the longitudinal axis.

In a preferred embodiment the protrusion is integrally and monolithic formed in the reservoir holder. That provides an easy and cost-saving manufacturing.

Furthermore, the cap is fixedly connected to a sterile barrier, for example a rigid needle shield, for the injection needle wherein upon cap removal the sterile barrier is removed from the injection needle together with the cap.

The holding member is preferably a deflectable arm or tongue and wherein in a deflected state of the arm or tongue the cap is released from the distal end portion.

The reservoir holder includes preferably a shoulder for supporting the reservoir and a proximal engagement portion to hold the reservoir immovably relative to the reservoir holder such that the reservoir cannot move relative to the reservoir holder before, during and after the injection.

### BRIEF DESCRIPTION OF THE DRAWINGS

The subject matter of the invention will be explained in more detail in the following text with reference to preferred exemplary embodiments which are illustrated in the attached drawings, in which:
- Fig. 1: depicts a perspective view of an autoinjector according to the invention;
- Fig. 2: depicts a perspective view of a syringe unit of the autoinjector;
- Fig. 3: depicts an exploded view of the syringe unit;
- Fig. 4: depicts a second embodiment of the syringe unit;
- Fig. 5: depicts a sectional view of a drive unit of the autoinjector;
- Fig. 6a: depicts a sectional view of the autoinjector with the syringe unit attached to the drive unit;
- Fig. 6b: depicts a sectional view of the autoinjector of figure 6a wherein the plane for the sectional view is offset and parallel a plane in the center of the longitudinal axis;
- Fig. 6c: depicts a sectional view of the autoinjector of figure 6a wherein the plane is offset and parallel the plane in the center of the longitudinal axis;
- Fig. 7: depicts the autoinjector with a syringe unit locked to the drive unit and without cap and a released cover sleeve before an injection;
- Fig. 8: depicts the autoinjector after a dispensing of a dose;
- Fig. 9a: depicts the drive unit without the outer shell parts and
- Fig. 9b, 9c: depict the upper and lower shell part of the drive unit.

The reference symbols used in the drawings, and their primary meanings, are listed in summary form in the list of designations. In principle, identical parts are provided with the same reference symbols in the figures.

### DETAILED DESCRIPTION OF PREFERRED EMBODIMENTS

In the present description the term "distal" refers to the side where the injection needle is located. This is on the left-hand side in the figures. The term "proximal" refers to the opposite side or rear end of the device and is on the right-hand side in the figures.

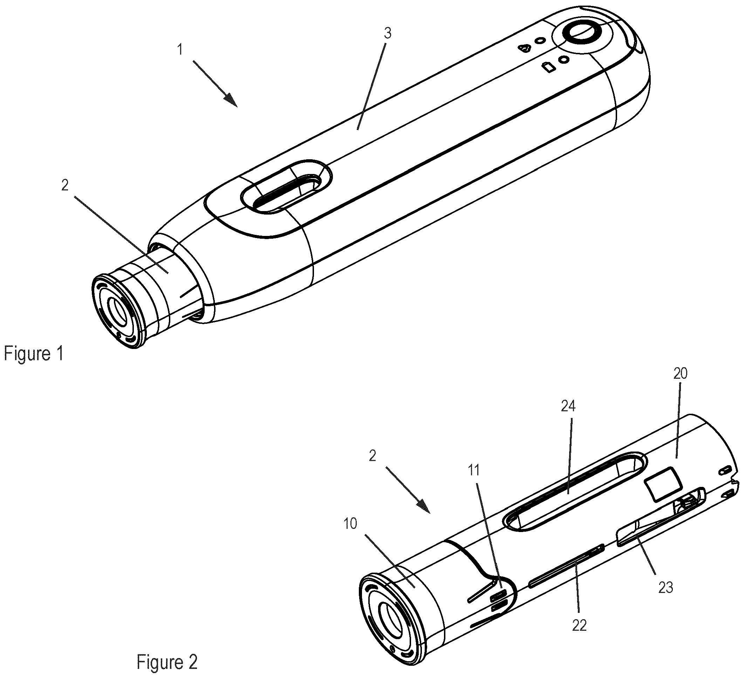

Figure 1 shows a perspective view of an injection device of a first embodiment of the invention in form of a semi-reusable autoinjector 1. The autoinjector 1 comprises a disposable reservoir unit in form of a syringe unit 2 and a reusable drive unit 3. As shown in figure 1 the drive unit 3 has an elongated housing defining a longitudinal axis and comprises a button 4 at its proximal end. The drive unit further includes an opening 81 or cavity adapted to accommodate a proximal portion of the syringe unit 2 to attach or couple the syringe unit 2 to the drive unit 3.

In the following the structural features of the syringe unit 2 and the drive unit 3 will be descripted in detail. Subsequently, the function of the semi-reusable autoinjector 1 will be explained.

### Syringe Unit

Figure 2 depicts a perspective view of the syringe unit 2 without the drive unit. The syringe unit 2 has an elliptical form in the cross section and extends along a longitudinal axis. On a distal end the cap 10 is mounted in an initial state or shipping state. The proximal end includes a holding structure as described below to releasably attach the syringe unit 2 to the drive unit 3.

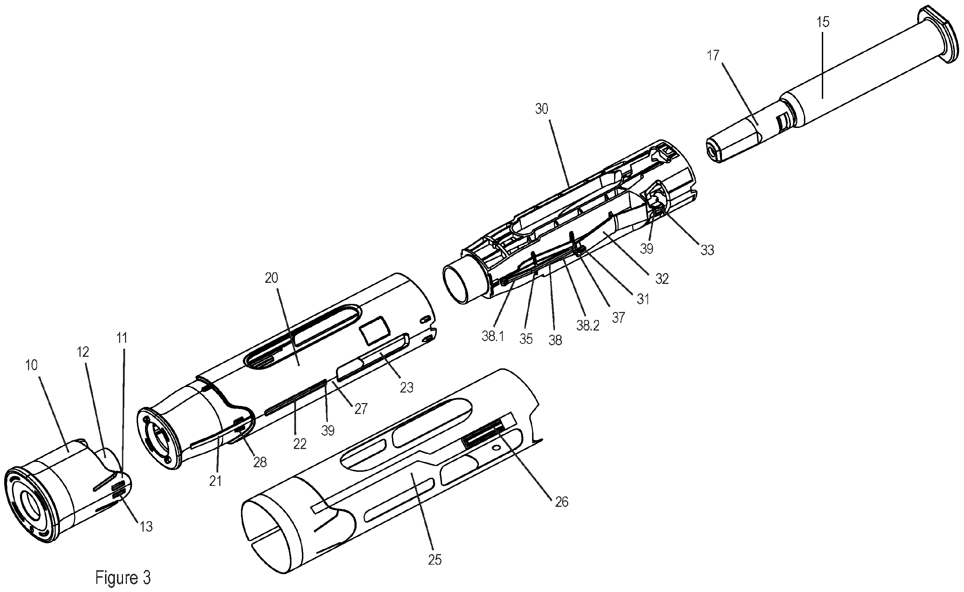

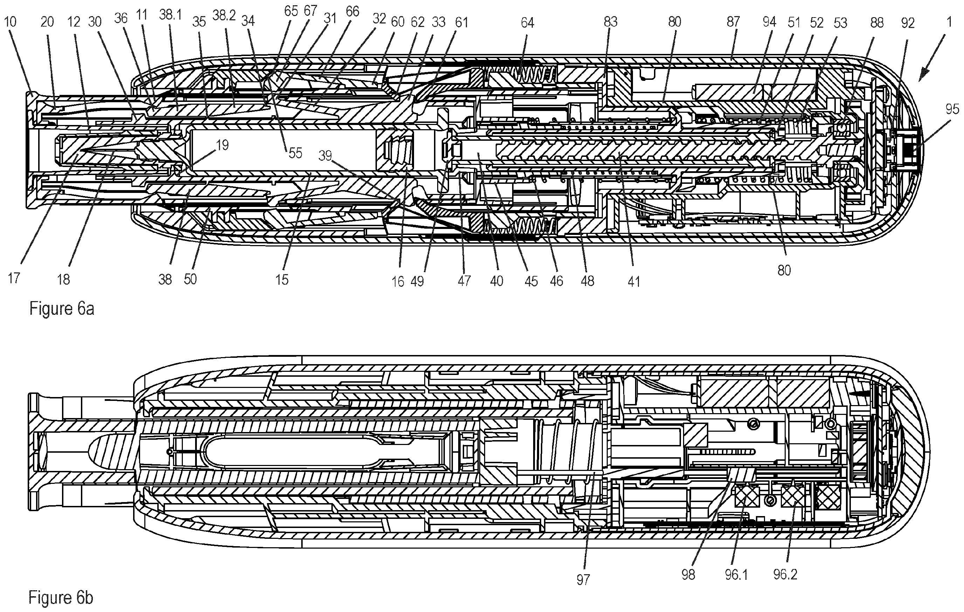

Figure 3 depicts an exploded view of the syringe unit 2. As shown in figure 3 the syringe unit 2 includes a syringe holder 30 (reservoir holder), a cover sleeve 20 coaxially arranged around the syringe holder 30 and movable relative thereto along the longitudinal axis, a label 25 wrapped around the cover sleeve 20, the cap 10 with a RNS remover and a prefilled syringe 15 (figure 6a) comprising a liquid drug and a rigid needle shield 17 (RNS) initially mounted on the injection needle. The syringe holder 30, the cover sleeve 20 and the cap 10 are individually injection-molded plastic parts which are all made of the same material, preferably polypropylene.

The prefilled syringe 15 with a barrel made of glass is rotationally and axially fixed inside the sleeve-shaped syringe holder 30. For that purpose, the syringe holder 30 includes clamping elements (not shown) pressing on an outer surface of the syringe barrel. The syringe holder 30 provides a bearing surface 19 (see figure 6a) on its inside adapted to abut a shoulder of the syringe barrel. The syringe holder 30 further features longitudinal guiding rails (not shown) on its outside for the cover sleeve 20 such that the cover sleeve 20 can be shifted exclusively in the longitudinal direction relative to the syringe holder 30.

As shown in figure 3 and 6a the syringe holder 30 includes two oppositely arranged and in longitudinal direction extending rockers 38 or seesaws. Each rocker 38 is pivotable around a center 35 which is formed by a flexible or elastic connection fixing the rocker 38 on an outer surface of the syringe holder 30. The center 35 divides the rocker 38 into a distal portion 38.1 or distal arm and a proximal portion 38.2 or proximal arm. The distal portion 38.1 is adapted to engage and deflect a cap holding tongue 11. For this purpose, the distal portion 38.1 includes a nose 36 (see figure 6a) adapted to engage a projection of the cap holding tongue 11 to disengage a snap-fit connection between tongue 11 and sleeve 20 or holder 30.

As best shown in figure 6a the proximal portion of the rocker 38 includes an actuation surface 34 adapted to be contacted by a free end of a release arm 32 (release member). The rocker 38 is adapted to be tilted or pivoted around the center 35 between a first or initial tilt position where the distal portion 38.1 is in a radially inward position and a second tilt position where the distal portion 38.1 is in a radially outward position.

The two radially deflectable release arms 32 (see figure 3 or 6a) are arranged on opposite sides of the syringe holder 30 and include each a distally directed free end with an end bulge 31 and a connecting end which connects the release arms 32 to the syringe holder 30. At the free end and lateral to the bulge 31 are two side support surfaces 37 on both sides of the bulge 31 to guide the release arms 32 as descripted below. The rocker 38 and the release arm 32 are integrally formed in the syringe holder 30 as a monolithic part. That means the syringe holder 30 with the rockers and the release arms is injection molded as one single part as best shown in figure 3.

In an initial state the release arms 32 are in a non-deflected state and in a locking position and prevent a movement of the cover sleeve 20 relative to the syringe holder 30 in a proximal direction. Hence, the cover sleeve 20 shields or covers the injection needle of the syringe 15 in a covering position. For that purpose, a free end of the release arms 32 abut stop surfaces (not shown) inside the cover sleeve 20. As will be descripted below once the release arms 32 are deflected radially inwards the cover sleeve 20 can be pushed proximally.

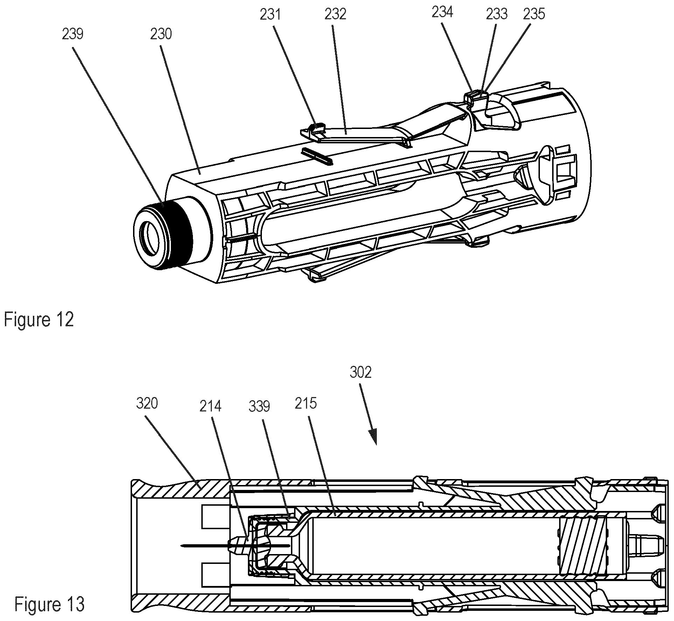

The syringe holder 30 further includes two oppositely arranged and radially protruding end ledges 33 or protrusions at a proximal end of the syringe holder 30 for engagement with clamping arms 61 (figure 6a) inside the drive unit 3. The protrusions have a rectangular cross section in a plane parallel to the longitudinal axis as best shown in figure 12 which depicts a perspective view of the syringe holder. As best shown in figure 12 the ledges 233 include a distal sloped surface 234 and a proximal sloped surface 235 to facilitate engagement with a clamp 62 of the clamping arm 61 and release or disengagement from the clamp 62. The first embodiment as shown in figures 1 to 8 may comprise the same shape of ledge 33 as the embodiment shown in figure 12. Each clamp 62 comprises on its distal side a sloped contact surface which further facilitates deflection of the clamping arm 61 upon insertion and subsequent engagement with the ledge 33.

As shown in figure 3 the cover sleeve 20 has a non-circular cross section and in particular an elliptical form in a plane perpendicular to the longitudinal axis. The cover sleeve comprises a medicament window 24 allowing the user to view the fill level of the syringe inside the syringe unit. The cover sleeve further includes a first lateral opening 21 for nose 36 of the rocker 38 to reach the cap holding tongues 11, a second opening 22 for access to the release arms 32 and a third opening 23 for access to the end ledges 33 from outside. The label 25 is wrapped around an outer surface of the syringe unit. The label includes a RFID code 26 integrated in the label and positioned in a proximal portion of the label 25. The code 26 includes information about the syringe unit for identification and may include further information about the drug inside the syringe, the volume, expire date and dispensing parameter such as injection speed and holding time The code 26 may have further country-specific information such as a user language to adjust a user interface language.

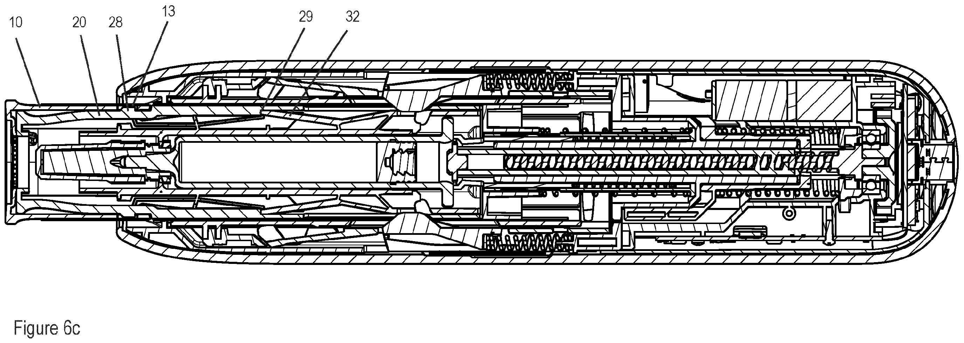

At a distal end of the cover sleeve 20 the cap 10 is releasably attached by a snap-fit connection. The cap 10 includes on two sides two deflectable holding members in form of holding tongues 11 on each side. The tongues 11 include on the free end an indentation or recess 13 adapted to be snapped to a protrusion 28 arranged on an outer surface of the cover sleeve 20 (also shown in figure 6c). Each tongue further includes a lobe projecting in the first opening 21 in the cover sleeve if the cap is mounted. The release of the snap-fit will be descripted below with respect to the decapping of the syringe unit 2. The cap 10 includes in its inside a sleeve-shaped holder 12 (RNS remover) connectable to the rigid needle shield (RNS) 17 of the syringe 15 such that the RNS 17 is removed with the cap 10 if the cap is released from the cover sleeve 20. The holder 12 is made of an elastomer and is shrunken onto the RNS during assembly of the syringe unit. As an outer cap housing is made of plastics the holder 12 and the outer cap housing from two-component injection molded part. The cap 10 with the holder are descripted in the patent application EP23165348.6 (Ypsomed) in detail which is incorporated herein by reference.

As an alternative the holder can be made of metal and coaxially arranged to the outer cap housing as the holder 12 descripted above. In this case the metal holder engages the RNS 17 and the RNS is removed together with the cap 10 if the cap is released from the needle cover.

The syringe 15 includes the syringe barrel and inside the barrel a movable piston 16 (figure 6a). The injection needle 18 (figure 6a) is non-releasably connected to a distal end of the syringe barrel. In an unused state the injection needle 18 is covered by the RNS 17, see figure 6a. The latter in turn is axially and radially fixedly connected to the cap 10 by the holder 12 as described above once the syringe 15 is mounted to the syringe holder 30.

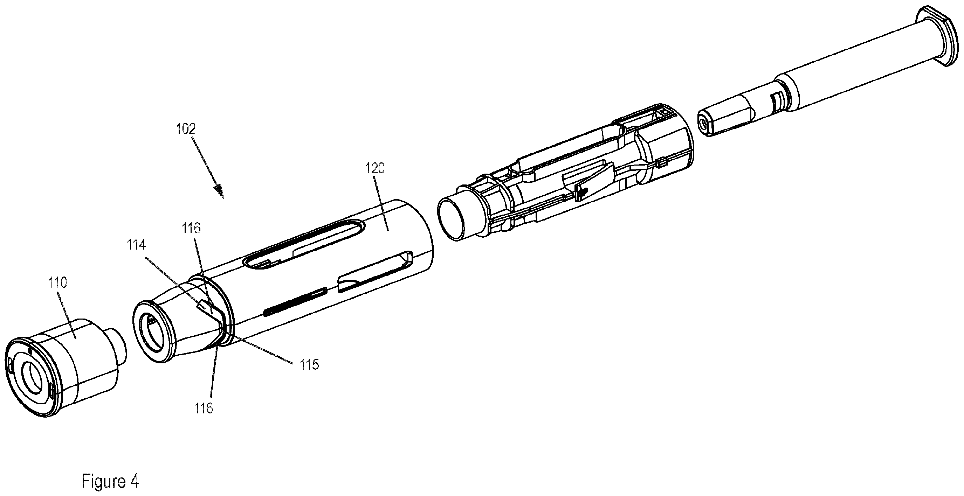

Figure 4 depicts an alternative syringe unit 102. In contrast to the syringe unit described with respect to figures 2 and 3 the syringe unit 102 shown in figure 4 includes a cap 110 mounted to the cover sleeve 120 by a bayonet-like connection.

Instead of the tongues the cap 110 includes two oppositely arranged and radially inward protruding cams or projections (not shown) adapted to project into corresponding grooves 114 in a distal cover sleeve portion.

The grooves 114 are U-shaped. Each groove 114 include a straight groove portion 115 which is perpendicular to the longitudinal axis and on both ends of the straight groove end groove portion 116 are arranged which are angled to the longitudinal axis.

The 110 cap can be mounted and released from the cover sleeve 120, respectively, by a combination of an axial and rotational movement. Hence, in contrast to the embodiment above with the rocker the cap 110 shown in figure 4 can be removed from the cover sleeve manually by a user force any time without a release mechanism with a rocker as described above.

If the user grabs the cap 110 and rotates the cap the cam enters the angled groove portions 116 and the cap 110 additionally moves axially in distal direction away from the cover sleeve end due to the angled groove portions 116. The user can then completely remove the cap 110 from the cover sleeve 120. All other features and functions of the syringe unit are the same as descripted with respect to the first embodiment shown in figure 3.

### Drive Unit

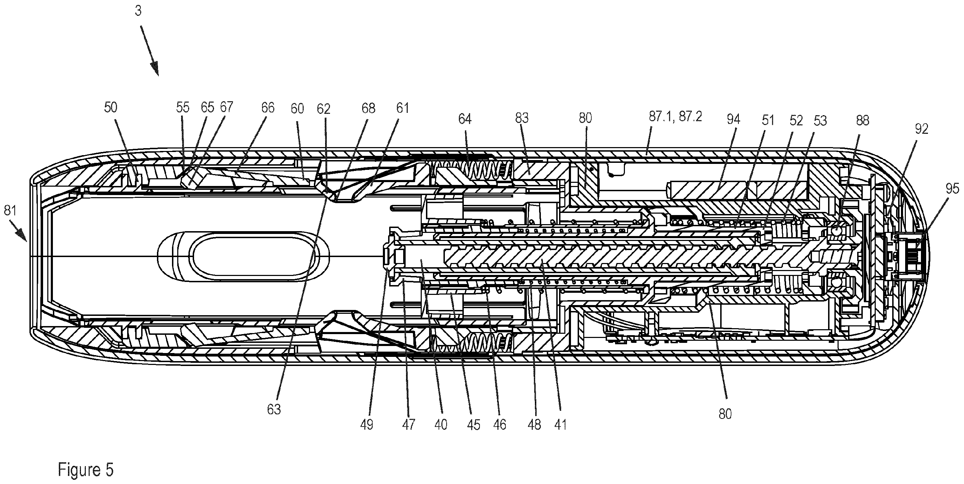

Figure 5 depicts a sectional view of the drive unit 3 without syringe unit. The cut of the sectional view runs along the longitudinal axis of the drive unit. The drive unit 3 comprises a support structure 80 including an outer housing cover or shell 87.1, 87.2 and inside the cover a mechanics sleeve 83 is fixedly connected to the support structure 80. Furthermore, the drive unit 3 includes the axial opening 81 on its distal side adapted to accommodate a proximal portion of the syringe unit 2.

The support structure 80 guides a gripper sleeve 60 which is immovably arranged. Coaxially and inside the gripper sleeve 60 a trigger sleeve 50 is arranged which is movable relative to the supporting structure 80 along the longitudinal axis. Again, inside the trigger sleeve 50 a movable cover sleeve connector 45 is located and biased in distal direction by a cover sleeve spring 46. The spring abuts on its distal end the cover sleeve connector 45 and on its proximal end a radial wall of the mechanics sleeve 83. A movable sleeve-shaped syringe connector 47 is coaxially arranged inside the cover sleeve connector 45 and biased distally by a syringe connector spring 48 coaxially inside the cover sleeve spring 46 and proximally supported by the mechanics sleeve 83. The spring 46 ensures that the syringe connector 45 pushes the syringe 15 of an inserted syringe unit 2 in distal direction.

In a proximal portion the support structure 80 supports and guides a sleeve-shaped trigger sleeve connector 51 movable along the longitudinal axis, a drive assembly with an electric motor (not shown) and a gear (not shown) connecting the motor with a threaded rod 41, a battery 94, the treaded rod 41 and a plunger rod 40 threadedly connected with the threaded rod 41. The support structure 80 further accommodates an electronic module 92 with a controller configured to control the electronic motor and providing information to the user via a display or LED 95 or a communication module (not shown). The trigger sleeve connector 51 is fixedly and immovably connected to the trigger sleeve 50 and distally pressed onto a proximal end of the plunger rod 40 by a trigger sleeve spring 53. The plunger rod 40 is non-rotatably guided by the mechanics sleeve 83 and comprises on its distal end a flange 49 adapted to engage the piston 16 (see figure 6a) during dispensing.

As shown in figure 6b inside the proximal portion of the support structure 80 a detection pin 97 is axially movable by the cover sleeve connector 45. The detection pin 97 includes a cam 98, which can interact with a position sensor implemented by two mechanical switches 96.1, 96.2 (figure 6b) arranged along the longitudinal axis. The presence of a syringe unit 2 inside the drive unit 3 is thus detectable via detection pin 97 and the first switch 96.1 which provides a corresponding signal to the controller of the electronic module. Furthermore, a retraction of the cover sleeve 20 can be detected by the second switch 96.2 as the cover sleeve 20 retraction further moves the pin 97 with its cam 98 in proximal direction.

Additionally, in a distal end portion a mechanical switch (not shown) held by the support structure 80 to sense the presence or absence of the cap 10 of an attached syringe unit 2.

The gripper sleeve 60 includes two axially extending and deflectable clamping arms 61 having a clamp 62 at their distal free end adapted to accommodate the syringe holder ledges 33. The clamping arms 61 can be deflected radially outwards allowing the clamps 62 to snap onto the ledges 33 or to release the ledges 33. As shown in figure 5 each clamp 62 includes a distally oriented sloped surface which facilitates the deflection of the clamping arm 61 upon contact with the syringe holder ledge 33. Moreover, each clamp 62 includes a proximally oriented holding surface 68 (figure 5) adapted to encounter a distal surface 39 (figure 3, 6a) of the ledge 33 when a syringe unit is attached to the drive unit. The holding surface 68 is radially offset of a center of rotation of the clamping arm 61 such that the clamping arm 61 reliably radially deflects when a force acts onto the holding surface 68 along the longitudinal direction and in distal direction. Furthermore, each clamping arm 61 provides a support for an axially aligned gripper sleeve spring 64 which biases the clamping arms 61 in a holding position.

Each clamping arm 61 includes a mechanical sensor with an actuation rod to detect when the syringe holder ledges 33 are inserted in the clamps 62. Alternatively, a strain gauge or a piezoelectric strain gauge may be arranged on a radial outer surface of the clamping arm 61 and electrically connected to the controller in the electronic module. Based on a strain gauge signal or piezoelectric sensor signal a deflection of the clamping arms 61 can be detected. This allows to determine if the clamping arms 61 were pivoted by the ledges 33 during insertion of the syringe unit 2 into the drive unit 3. That in turn allows to conclude if a syringe unit is completely inserted into and attached to the drive unit.

As a further alternative light guides (fiber optics) may be used to detect the clamping arm deflection. That means a light guide is arranged from the PCB to each clamping arm 61 such that a deflection of the clamping arms 61 interrupts the light guide. The corresponding signal allows to determine that the clamping arms were deflected by the ledge 33 of the inserted syringe unit 2.

The gripper sleeve 60 further includes two oppositely arranged and radially inward deflectable actuation arms 66. The actuation arms 66 extend essentially in the longitudinal axis and include on their distal free end 67 a cam with a guiding surface 65 or sloped contact surface. The actuation arms 66 are deflectable radially inwards upon contact with a counter guiding surface 55 or counter sloped contact surface of the trigger sleeve 50. The free ends 67 of the actuation arms 66 further comprise each a radially inward directed contact surface to abut the end bulge 31 of the release arm 32 of the syringe unit 2 as described further below. On a proximal side the cams include a locking surface adapted to engage a distal surface of the syringe unit as described below.

The trigger sleeve 50 is axially guided by support structure 80 and shiftable relative thereto. For this purpose, the trigger sleeve 50 includes longitudinal guiding rails (not shown) engaging longitudinal grooves (not shown) in support structure. As it can be seen in figure 5 and 6a the trigger sleeve 50 incudes oppositely arranged contact elements featuring the counter guiding surface 55 or counter sloped contact surface. As mentioned above the trigger sleeve 50 is further immovably connected to the trigger sleeve connector 51. As the trigger sleeve connector 51 is biased by the trigger sleeve spring 53 the trigger sleeve 50 is biased too in distal direction. The trigger sleeve connector 51 in turn is connected to the plunger rod via ledges 52 which engage a proximal shoulder of the plunger rod 40 such that an axial movement of the plunger rod 40 in proximal direction also moves the trigger sleeve 50 proximally.

In the state shown in figure 5 the drive unit 3 is ready to be loaded with a syringe unit 2. The controller displays the current state to the user by means of a steady green light.

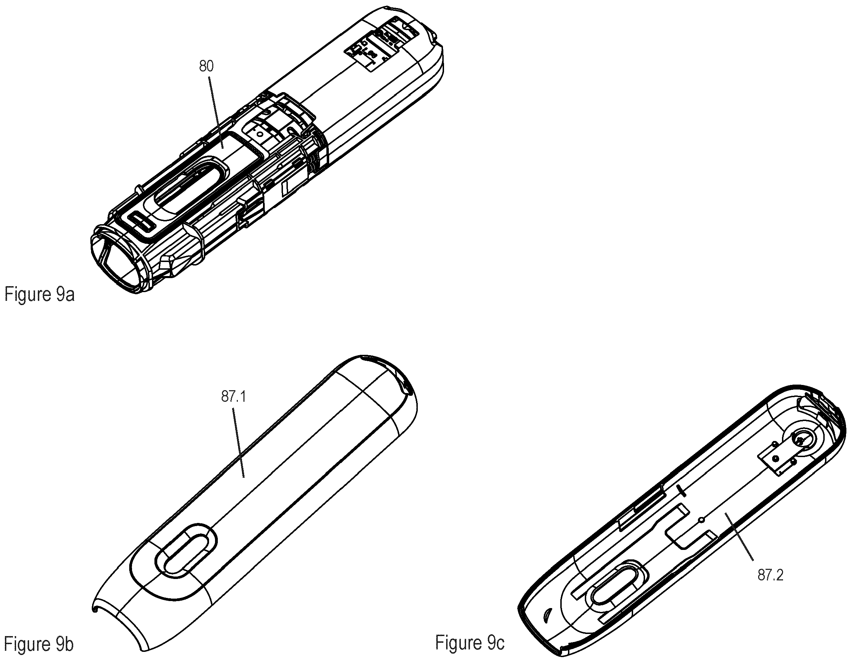

The support structure 80 supports and holds the whole drive mechanism including the above-mentioned trigger sleeve 50 and gripper sleeve 60 as well as the motor and the electronic module in a proximal portion. The drive unit 3 further includes an outermost shell comprising two shell parts 87.1, 87.2 that are connected to each other by a snap-fit connection so that the parts are tightly fixed to each other. The support structure with the drive mechanism is depicted in figure 9a whereas the upper shell part 87.1 is shown in figure 9b and the lower shell part 87.2 in figure 9c.

The shell parts do not support any internal component but are fixedly connected to the support structure 80 and act as a grip for the user. The shell parts 87.1, 87.2 may be adjusted in color, in the form, size and appearance for a specific use case.

### Function

In order to prepare the autoinjector 1 for an injection the user attaches a syringe unit 2 to the drive unit 3. The electronics in the drive unit 3 are switched from an inactive state or sleep mode to an active mode upon detection of the syringe unit by the switch 96.1 (figure 6b) of the position sensor. Alternatively, the user can press the button 4 to activate the electronics.

Figure 6a, 6b and 6c depict sectional views of the drive unit 3 with an inserted syringe unit 2. In figure 6a the cut for the sectional view runs along the longitudinal axis in a center plane. The plane of the section for Figure 6b and 6c is parallel but in a radial distance to a center longitudinal axis and hence offset from and parallel to the center plane.

In the state shown in figures 6a, 6b and 6c the syringe unit 2 has been inserted with its proximal end portion into the distal opening of the drive unit 3. In the state shown in figures 6a, 6b and 6c the proximal ledges 33 of the syringe holder 30 are snapped into the clamps 62 of the gripper sleeve clamping arms 61. The syringe unit 2 is thus held in the drive unit 3 by the clamping arms 61. However, in the state in figures 6a, 6b and 6c the syringe unit 2 can still be pulled out of the drive unit 3 by overcoming a holding force. Hence, if the user pulls the syringe unit 2 in a distal direction the clamping arms 61 are deflected radially. The ledges 33 of the syringe holder 30 are therefore released from the clamps 62 and the syringe unit 2 can be removed from the drive unit 3.

The presence of the ledges 33 in the clamps is detected by the controller as the ledges abut a cam of the mechanical switch inside the clamps 62. The controller thus receives a signal that a syringe unit is correctly and completely inserted into the drive unit. Alternatively, if the detection is provided by a strain gauge on the clamping arms 61 or a light guide interrupted by the deflecting clamping arms 61 an engagement movement of the clamping arms is detected and hence the controller can determine that a syringe unit 2 is attached based on the strain gauge or light guide signal.

That means in this state the syringe unit 2 is held inside the drive unit 3 but not yet locked inside the drive unit 3. The mechanical switch in the distal end portion of the support structure 80 is actuated by the cap 10 of the inserted syringe unit. Hence, the controller can determine that a cap 10 is mounted on the inserted syringe unit 2.

In the initial state or shipping state the cover sleeve 20 is locked into the covering position. This is shown in figure 6c where the plane for the sectional view is offset a center axis. As it can be seen in figure 6a a distally oriented end of the release arm 32 abut or engage a proximally oriented stop surface 29 of the cover sleeve 20. A force in proximal direction acting on the cover sleeve 20 is thus absorbed by the release arm 32 and hence the cover sleeve cannot be moved out of the covering position towards the retracted position.

Furthermore, in an initial state or shipping state of the syringe unit the syringe barrel does not abut the bearing surface 19 and hence between a distal end of the syringe barrel and the bearing surface 19 of the syringe holder 30 is a gap. The gap may be several millimeters, in particular 2 to 5 mm.

The reason for the presence of the gap is that the device cap 10 has to be movable a short distance if the user grabs the cap 10 to remove the (unused) syringe unit or to position the syringe unit. The travel of the cap is due to a play provided between the parts to account for manufacturing or assembly tolerances. During such a cap movement a removal of the RNS 17 and thus a removal of the sterile barrier is not desired.

As there is an initial gap between the syringe barrel and the bearing surface 19 the RNS (with the needle) can travel a short distance without impact to the sterile barrier. That means the gap between the syringe barrel and the syringe holder bearing surface 19 is preferably larger than a maximal play of the cap (cap travel relative to the syringe holder).

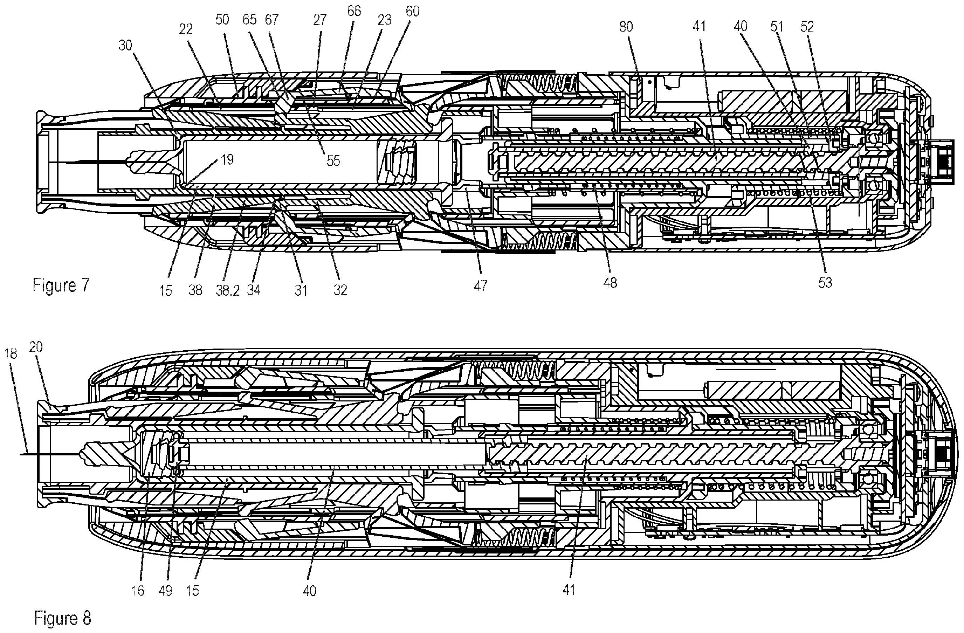

Figure 7 depicts the autoinjector 1 in a locked state without cap. When the syringe unit 2 is inserted as described above the proximal end of the syringe unit 2 moves the detection pin 97 proximally and thus the switch 96.1 of the position sensor automatically sends a signal to the controller in the electronic module which detects the presence of the syringe unit 2 inside the drive unit 3. The controller wakes up the electronics and sets the device in an active state. A code reader (not shown) in form of an NFC reader inside the drive unit 3 reads the RFID code 26 of the syringe unit 2. Optionally, a temperature sensor is additionally integrated in the syringe unit RFID code configured to sense the current syringe unit temperature. Subsequently, the controller of the drive unit sends the read medication information and eventually the temperature information from the read code 26 to an external device to verify the drug or medication (and eventually the temperature information) with predefined values or tables. Alternatively, the controller compares the read information from the code 26 with predefined information stored in a memory inside the drive unit 2.

In case of successful medication and eventually temperature verification the controller receives an enable signal (either from the external device or from the internal comparison). The controller then controls the electric motor to rotate the threaded rod 41 to move the trigger sleeve 50 a short distance of several millimeters in proximal direction. Such a trigger sleeve 50 movement is achieved by a kinematic chain as described as follows.

The motor rotates a pinion and the gear transfers the rotation to the threaded rod 41 which in turn moves the non-rotating plunger rod 40 in proximal direction, preferably an axial travel of about 5 mm. As the trigger sleeve connector 51 is connected via its ledges 52 to a proximal shoulder of the plunger rod 40 the latter causes the trigger sleeve connector 51 to move and thus the trigger sleeve 50 is shifted in proximal direction too. Furthermore, the trigger sleeve spring 53 is compressed during the proximal movement.

The proximal movement of the trigger sleeve 50 brings the counter sloped guiding surfaces 55 of the trigger sleeve 50 into contact with the sloped guiding surfaces 65 of the actuation arms. As the two sloped surfaces 65, 55 slide along each other the actuation arms 66 are forced to deflect radially inwards and the actuation arms free ends 67 are pressed radially under the trigger sleeve part with the guiding surface 65 as shown in figure 7 when the trigger sleeve 50 is further moved in proximal direction. The radial inward directed contact surface of the free ends 67 of the deflected actuation arms in turn encounter the end bulges 31 of the release arms 32 thereby deflect the release arms 32 radially inwards too such that the release arms 32 are in a release position. Subsequently, the inwardly moving release arms 32 abut the rocker actuation surface 34 and tilt the rocker 38 from the first tilt position towards the second tilt position such that the proximal portion 38.2 of the rocker with the actuation surface 34 is pivoted radially inwards.

Consequently, the distal portion 38.1 of the rocker moves radially outwards and hence deflects the holding tongues 11 of the cap 10 radially outwards. That in turn means that the snap-fit connection holding the cap 10 onto the distal end of the cover sleeve 20 is released as the recesses 13 of deflected holding tongues 11 no longer engage the protrusions 28 on the cover sleeve 20. The user can thus pull off the cap 10 form the autoinjector.

When the cap 10 is removed the cam of the mechanic switch is no longer actuated and hence the controller derives from the corresponding switch signal that the cap 10 is removed. Alternatively, if the sensor is implemented with a light guide the removal of the cap 10 interrupts the light guide or alternatively allows an uninterrupted light guide and hence a corresponding signal indicates that the cap is no longer present. Additionally, in case a light guide is used the cap can be illuminated by the light guide if the cap is made of a translucent or transparent plastic. That means the controller may activate a light flashing or constantly illuminating the cap in a colour signaling the user that the cap is released and can be removed. If the cap is removed the light guide is interrupted and a cap removal signal is generated for the controller.

If the cap 10 is mounted the syringe 15 inside the syringe holder 30 is hold in place by the cap 10 via holder 12 and RNS 17 and thus the distally biased syringe connector 47 cannot move the syringe 15 relative to the syringe holder 30. However, when the cap 10 is removed the syringe 15 is no longer hold and due to the syringe connector spring 48 the syringe connector 47 moves the syringe 15 relative to the syringe holder 30 distally and thus the gap between the distal end of the syringe barrel and the bearing surface 19 disappears. That means upon cap removal the syringe connector urges the syringe 15 against the bearing surface 19 such that the syringe 15 is fixedly hold between the biased syringe connector 47 and the bearing surface 19.

The radially inwardly deflected release arms 32 have further the effect that they no longer engage the stop surface 29 (figure 6c) of the cover sleeve 20 and thus allow for the cover sleeve 20 to move out of a covering position and move inside the support structure 80 in proximal direction relative to the syringe holder 30.

At the same time the radially inwards deflected actuation arms 66, namely a proximally oriented locking surface of the free ends 67 of the actuation arms 66 abut a distal surface of a rib 27 of the cover sleeve 20. The rib 27 is located between the opening 22 and opening 23 in the cover sleeve shown in figure 3 and in the sectional view in figure 6c. That means in this locked state the syringe unit 2 cannot not be pulled out as the free end 67 abuts the distal surface of the rib 27 of the cover sleeve and hence the syringe unit 2 is locked in the drive unit.

The cap 10 of the autoinjector 1 is now removed and the autoinjector is ready for an injection. The controller displays a corresponding notification on the display the LED 95 constantly lights.

As a next step the user places the distal end of the cover sleeve 20 onto an injection site and pushes the autoinjector 1 towards the injection site. This causes the cover sleeve 20 to move proximally from the distal covering position into support structure 80 (push on skin) and compresses the cover sleeve spring 46 which biases the cover sleeve 20 in distal direction via cover sleeve connector 45. The retraction of the cover sleeve 20 is possible as the end ledges 31 of the release arms 32 are hold radially inwards by the deflected actuation arms 66 (figure 7). When the cover sleeve 20 is in its retracted position the injection needle 18 protrudes from the cover sleeve as shown in figure 8.

Upon push on skin the cover sleeve connector 45 is moved proximally and thus the detection pin 97 is further moved proximally. The further movement brings the cam 98 in contact with the second switch 96.2 (figure 6b) which triggers the controller in the electronic module to start the injection. That means the controller drives the electric motor in dispensing direction and thus rotates the threaded rod 41 and thereby moves the plunger rod 40 in distal direction.

The controller drives the plunger rod distally with a low torque. Upon contact of the plunger rod flange 49 with the piston 16 inside the syringe barrel the motor current increases due to the motor controlling. That is a signal for the controller that the plunger rod 40 is correctly positioned. Subsequently, the motor drives the plunger rod to dispense the predefined dose.

During dispensing the distal flange 49 of the plunger rod 40 moves the piston 16 inside the syringe 15 in distal direction and thus dispenses liquid drug through the injection needle out of the syringe 15. If the piston 16 abuts a distal end inside the syringe barrel the current of the motor controlling increases and the controller stops to drive the plunger rod distally. This end of injection condition is shown in figure 8. As it can be seen the plunger rod 40 and thus the piston 16 inside the syringe barrel is in a distal end position.

The distal movement of the plunger rod 40 moves the trigger sleeve 50 via trigger sleeve connector 51 in distal direction to its initial position. That means the actuation arms 66 can move radially outwards back into their non-deflected position. However, as the cover sleeve 20 is in its retracted proximal position the release arms 32 are prevented from being moved back into the initial non-deflected position by the side support surfaces 37 which abut on an inner surface of the retracted cover sleeve. That means as long as the cover sleeve 20 is in its retracted position the release arms 32 remain deflected.

The user may interrupt the dispensing movement by pressing the button 4. The controller stops the motor and thus the plunger rod movement. The dispensing can be continued when the button 4 is pressed again.

The controller displays a holding time indicating a period during that the user has to hold the autoinjector 1 onto the injection site after the injection to ensure that the drug is completely administered and absorbed. The controller may display a down counter informing the user about the remaining holding time. The counter is implemented by a progression bar provided by a display visible from outside. Furthermore, the controller can drive the motor without moving the piston rod at idle speed to generate a motor sound which should prevent the user to remove the autoinjector from the injection site too early.

Once the holding time is elapsed a notification in form of a sound is emitted indicating the user that the autoinjector can be removed from the injection site. Alternatively, the end of the holding time may be indicated only by a LED.

When the user removes the autoinjector 1 from the injection site compressed cover sleeve spring 46 can release and thus it can move the cover sleeve 20 back distally into the needle covering position.

Upon movement of the cover sleeve 20 into its covering position the side support surfaces 37 get out of contact with the inner surface of the cover sleeve 20 and the release arms 32 can pivot radially outwards back into their locking position in which the free end of the arms 32 abut the contact surface inside the cover sleeve 20 thereby locking the cover sleeve 20 in its covering position. Hence, after the injection process the cover sleeve 20 cannot be moved into the retracted position again.

Upon completion of the injection the controller sends recorded injection data with a time stamp to an external receiver, e. g. a cloud server.

Furthermore, as the actuation arms 66 are not deflected anymore and thus do not contact the distal stop surface of the syringe holder 30 the entire syringe unit 2 can be pulled out of the drive unit by the user. The user can now discard the syringe unit 2 and the drive unit 3 is ready to be loaded with a new syringe unit.

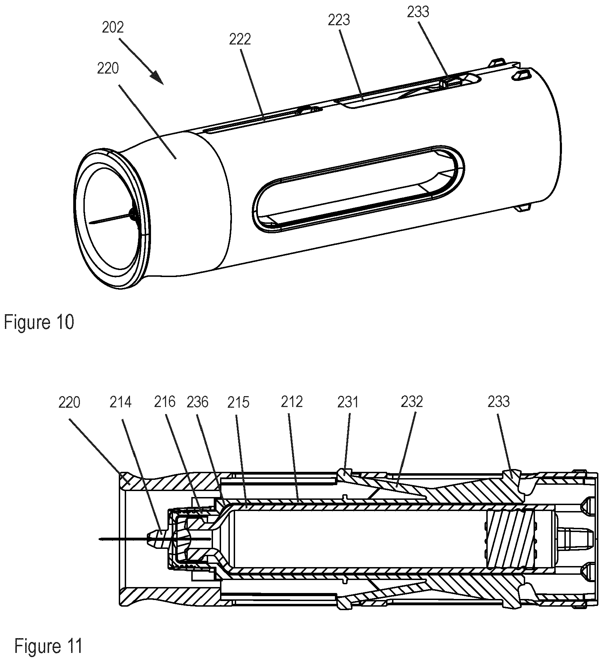

Figure 10 depicts a further embodiment of the present invention. In contrast to the first embodiment the reservoir unit in this embodiment is a cartridge unit 202 comprising a cartridge 215 instead of a syringe.

In contrast to a syringe the cartridge 215 does not include a permanently attached injection needle. A cartridge holder 230 comprises a needle mounting portion 239 on a distal end for releasable attachment of an injection needle assembly 214. That means the user has to mount the needle assembly 214 to the distal end of the cartridge holder 230 prior injection.

Figure 11 depicts a sectional view of the cartridge unit 202. The cartridge holder 230 is adapted to hold the cartridge 215 such that it can not move relative to the holder. The needle mounting portion 239 forms a recess or opening adapted to accommodate a distal portion of the cartridge or a crimp of the cartridge holder comprises further on an inside a shoulder 236 which supports a distal shoulder of the cartridge 215 to axially hold the cartridge in the distal direction. In a proximal portion the cartridge holder 230 comprises a support element that presses on the cartridge to fix the cartridge and to prevent a proximal movement of the cartridge 215 relative to the holder 230. Therefore, the cartridge holder 230 holds the cartridge 215 immovably such that the cartridge 215 cannot displace relative to the holder 230. The needle mounting portion 239 comprises circumferential grooves or a thread for attaching the needle assembly 214.

As shown in figure 10 and 11 the cartridge unit 202 comprises a cover sleeve 220 with a reduced length compared to the first embodiment in the longitudinal direction allowing access to the needle mounting portion 239. The cover sleeve 220 in this embodiment is exclusively used to detect injection site contact and to start an injection when the user presses the injection device against the injection site (push on skin).

The cartridge holder 230 with its end ledges 233 for coupling the cartridge unit 202 to the drive unit and its release arms 232 with end bulges 231 has the same function as described above with respect to the first embodiment. That means the cartridge unit 202 can be used with the drive unit 3 of the first embodiment and the functions of injection process as described above also apply to the cartridge unit 202 with the cartridge 215.

Figure 12 depicts a perspective view of the cartridge holder 230 alone. As shown in this figure the protrusion or ledge 233 comprises a distal sloped surface and a proximal sloped surface 235 adapted to facilitate insertion into the clamps of the clamping arms of the drive unit 3 or release and disengagement of the clamps as described with respect to the first embodiment.

Figure 13 depicts a sectional view of a further embodiment of a cartridge unit 302 comprising a cartridge 215. In comparison with the embodiment shown in figures 10 and 11 the version shown in figure 13 comprises a prolonged cover sleeve 320 allowing to cover the injection needle of a mounted needle assembly 214 when the needle cover 320 is in a covering position. For this version, the prolonged needle cover 320 requires a specific tool to enable the user to mount the needle assembly 214 as the needle cover completely covers a distal portion of the cartridge holder with its needle mounting portion.

While the invention has been described in detail in the drawings and foregoing description, such description is to be considered illustrative or exemplary and not restrictive. Variations to the disclosed embodiments can be understood and effected by those skilled in the art and practising the claimed invention, from a study of the drawings, the disclosure, and the appended claims. In the claims, the word "comprising" does not exclude other elements or steps, and the indefinite article "a" or "an" does not exclude a plurality. The mere fact that certain elements or steps are recited in distinct claims does not indicate that a combination of these elements or steps cannot be used to advantage, specifically, in addition to the actual claim dependency, any further meaningful claim combination shall be considered disclosed.

**LIST OF DESIGNATIONS**

| | | | | | |
|---|---|---|---|---|---|
| 1 | autoinjector | 50 | trigger sleeve | 202 | cartridge unit |
| 2 | syringe unit | 51 | trigger sleeve connector | 214 | needle assembly |
| 3 | drive unit | | | 215 | cartridge |
| 4 | button | 52 | ledge | 220 | cover sleeve |
| | | 53 | trigger sleeve spring | 222 | second opening |
| 10 | cap | 55 | counter sloped guiding surface | 223 | third opening |
| 11 | holding tongue | | | 230 | cartridge holder |
| 12 | holder | 60 | gripper sleeve | 231 | end bulge |
| 13 | recess | 61 | clamping arms | 232 | release arm |
| 15 | prefilled syringe | 62 | clamp | 233 | end ledge |
| 16 | piston | 63 | sloped surface | 234 | distal sloped surface |
| 17 | rigid needle shield RNS | 64 | gripper sleeve spring | 235 | proximal sloped surface |
| 18 | injection needle | 65 | sloped guiding surface | | |
| 19 | bearing surface | 66 | actuation arm | 236 | shoulder |
| | | 67 | free end | 239 | needle mounting portion |
| 20 | cover sleeve | 68 | holding surface | | |
| 21 | first opening | | | | |
| 22 | second opening | 80 | support structure | 302 | cartridge unit |
| 23 | third opening | 81 | axial opening | 320 | cover sleeve |
| 24 | medicament window | 83 | mechanics sleeve | 339 | needle mounting portion |
| 25 | label | 87.1 | first shell | | |
| 26 | RFID tag | 87.2 | second shell | | |
| 27 | rib | 88 | bearing | | |
| 28 | protrusion | 92 | electronics | | |
| 29 | stop surface | 94 | battery | | |
| | | 95 | LED | | |
| 30 | syringe holder | 96.1 | first switch | | |
| 31 | end bulge | 96.2 | second switch | | |
| 32 | release arm | 97 | pin | | |
| 33 | end ledge | 98 | cam | | |
| 34 | actuation surface | | | | |
| 35 | centre | 102 | syringe unit | | |
| 36 | nose | 110 | cap | | |
| 37 | side support surface | 114 | groove | | |
| 38.1 | distal portion | 115 | straight portion | | |
| 38 | rocker | 116 | angled portion | | |
| 38.2 | proximal portion | 120 | cover sleeve | | |
| 39 | distal surface | | | | |
| 40 | plunger rod | | | | |
| 41 | threaded rod | | | | |
| 45 | cover sleeve connector | | | | |
| 46 | cover sleeve spring | | | | |
| 47 | syringe connector | | | | |
| 48 | syringe connector spring | | | | |
| 49 | flange | | | | |

## Claims

1. A method for releasably attaching and locking a reservoir unit (2) to a drive unit (3) of an injection device (1), wherein the injection device includes a cap (10) attachable to a distal end portion of the reservoir unit (2) and wherein the reservoir unit (2) is adapted to hold a reservoir (15) and includes
- an outer cover (20) with a lateral opening (22);
- a release member (32) movable relative to the cover (20) wherein the release member (32) can be in a locking position in which the cap (10) is locked to the reservoir unit (2) and in a release position in which the cap is released,
and wherein the drive unit (3) comprises
- a housing (87.1, 87.2) defining a longitudinal axis and including an axial opening (81);
- an actuation arm (66) movable relative to the housing and having a locking portion (67) on a free end and
- a trigger member (50) movable along the longitudinal axis relative to the housing,
wherein the method comprises the steps of
a. Inserting at least a proximal portion of the reservoir unit (2) into the axial opening (81);
b. Moving the trigger member (50) along the longitudinal axis;
c. Moving the actuation arm (66), by the trigger member (50) movement, from a radial outer position into a radial inner position and thereby inserting the locking portion (67) into the lateral opening (22) such that locking portion prevents a distal retraction movement of the reservoir unit (2) and locks the reservoir unit (2) to the drive unit (3) and
d. Engaging, by the locking portion (67) in its inner position, the release member (32) and thereby moving the release member (32) into its release position or holding the release member in its release position such that the cap (10) can be removed from the reservoir unit (2).

2. Method according to claim 1, wherein the insertion includes a coupling of a connecting portion (62) of a movable clamping arm (61) of the drive unit (3) to a radially extending protrusion (33) of the reservoir unit (2) to releasably couple the reservoir unit (2) to the drive unit (3).

3. Method according to claim 2 wherein the coupling includes a radial deflection of the clamping arm (61) by the protrusion (33) and by a sloped surface (63) of the connecting portion (62) upon insertion of the reservoir unit (2) into the drive unit (3).

4. Method according to claim 2 or 3, wherein during the coupling the protrusion (33) enters a clamp or recess of the connecting portion (62) and establishes a snap-fit connection to releasably couple the connecting portion (62) to the protrusion (33).

5. Method according to claim 4, wherein the clamp or recess includes a proximally oriented holding surface (68) encountering the protrusion (33) when the reservoir unit (2) is attached to the drive unit (3) and wherein the holding surface (68) is radially offset of a center of rotation of the clamping arm (61) such that the clamping arms radially deflects when a force acts onto the holding surface along the longitudinal direction and in distal direction.

6. Method according to any of claims 2 to 5, wherein the protrusion (33) has a rectangular-shaped cross section in a plane parallel to the longitudinal axis.

7. Method according to any of claims 1 to 6, wherein the locking portion (67) includes a radially inwards protruding cam comprising a proximally oriented locking surface which encounters a distal surface (39) of the reservoir unit (2) when a force acts on the reservoir unit in distal direction.

8. Method according to claim 7, wherein the lateral opening (22) has an elongated form extending along the longitudinal axis and wherein the distal surface (39) defines a proximal end of the lateral opening (22).

9. Method according to any of claims 1 to 8, wherein the outer cover (20) is a needle cover which is the outermost part of the reservoir unit (2) and wherein the needle cover (20) is movable along the longitudinal axis relative to an injection device housing between a needle covering position, in which the needle is covered and a retracted position, in which the needle is exposed.

10. Method according claim 9, wherein the reservoir unit (2) comprises a lock element (31) movable relative to the cover (20) wherein the lock element (31) can be in a locking position in which the lock element engages the needle cover (20) and prevents the needle cover from being moved out of the covering position and the lock element (31) can be in an unlocking position in which it does not engage the needle cover and does not prevent the needle cover from being moved out of the covering position.

11. Method according to any of claims 1 to 10, wherein moving the release member (32) into its release position includes a tilting of a rocker (38) by actuating a first rocker portion (38.2) and thereby tilting the rocker from a first or initial tilt position of the rocker (38) in which a deflectable holding member (11) holds the cap on the distal end to a second tilt position which is different from the first tilt position and in which a second rocker portion (38.1) deflects the holding member (11) such that the cap (10) is released and can be removed from the distal end portion.

12. Method according to claim 11, wherein the rocker (38) is tiltable around an axis perpendicular to the longitudinal axis and wherein a center of the rocker divides the rocker in the first rocker portion (38.2) and in the second rocker portion (38.1).

13. Use of a reservoir unit (2) adapted to hold a reservoir containing the liquid drug, the reservoir unit (2) comprising a lateral opening (22) for locking the reservoir unit (2) to the drive unit (3) according to the method claims 1 to 12.

14. Use according to claim 13, wherein the reservoir unit is a syringe unit (2) comprising a prefilled syringe (15) with an injection needle (18) permanently attached to a syringe barrel and wherein the syringe unit further includes a syringe holder (30) adapted to immovably hold the syringe (15).

15. Use according to claim 13, wherein the reservoir unit (202) is a cartridge unit comprising a cartridge (215) with a needle mounting portion (239) on a distal end for releasable attachment of an injection needle assembly (214) and wherein the cartridge unit (202) further includes a cartridge holder (230) for immovably hold the cartridge (215).c
